(19) **Europäisches Patentamt / European Patent Office / Office européen des brevets**

(11) **EP 4 660 294 A1**

(12) **EUROPEAN PATENT APPLICATION**
published in accordance with Art. 153(4) EPC

(43) Date of publication:
**10.12.2025 Bulletin 2025/50**

(21) Application number: **24749688.8**

(22) Date of filing: **30.01.2024**

(51) International Patent Classification (IPC):
**C12N 1/20** (2006.01)   **A61K 35/747** (2015.01)
**A61P 31/04** (2006.01)   **A23L 33/135** (2016.01)
**C12R 1/225** (2006.01)

(52) Cooperative Patent Classification (CPC):
**A23L 33/135; A61K 35/747; A61P 1/00; A61P 1/10;
A61P 1/12; A61P 3/04; A61P 3/06; A61P 3/10;
A61P 9/12; A61P 11/02; A61P 19/06; A61P 19/10;
A61P 25/20; A61P 25/22; A61P 25/24;**   (Cont.)

(86) International application number:
**PCT/CN2024/074770**

(87) International publication number:
**WO 2024/160209 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.02.2023   CN 202310050530**

(71) Applicant: **Beijing Sanyuan Foods Co., Ltd.
Beijing 100163 (CN)**

(72) Inventors:
• **CHEN, Lijun
   Beijing 100163 (CN)**
• **LI, Xianping
   Beijing 100163 (CN)**

• **LIU, Lu
   Beijing 100163 (CN)**
• **LIU, Bin
   Beijing 100163 (CN)**
• **ZHAO, Junying
   Beijing 100163 (CN)**
• **QIAO, Weicang
   Beijing 100163 (CN)**
• **JI, Yadong
   Beijing 100163 (CN)**
• **HUANG, Jiahao
   Beijing 100163 (CN)**

(74) Representative: **LLR
   2, rue Jean Lantier
   75001 Paris (FR)**

(54) **BREAST MILK-DERIVED LACTICASEIBACILLUS RHAMNOSUS AND USE**

(57)   Provided are a breast milk-derived Lacticaseibacillus rhamnosus and a use thereof. Lacticaseibacillus rhamnosus B2-1 was preserved in the China General Microbiological Culture Collection Center (CGMCC) on 08 December 2022, and has an accession number of CGMCC NO.26167. Also provided are a use of the Lacticaseibacillus rhamnosus in preparation of a drug and/or food and a composition comprising the Lacticaseibacillus rhamnosus and/or a fermentation product thereof. The Lacticaseibacillus rhamnosus is separated from breast milk, and is more easily accepted by consumers than other sources of lactobacilli; in addition, the strain also has good acid resistance, bile salt resistance, and bactericidal and immunomodulatory properties, and has good application prospects in the fields of drugs and foods.

Figure 1

**(Cont. next page)**

(52) Cooperative Patent Classification (CPC): (Cont.)
**A61P 25/28; A61P 29/00; A61P 31/04;
A61P 37/02; C12N 1/20;** C12R 2001/225

**Description**

**Cross-Reference to Related Application**

The present application claims priority to a Chinese patent application filed with the China National Intellectual Property Administration on February 1, 2023, with application number 202310050530.6 and title "Breast Milk-Derived *Lacticaseibacillus Rhamnosus* and Use". The entire content of this application is incorporated herein by reference.

**Technical Field**

The present application relates to the field of microorganisms, and in particular to a strain of *Lacticaseibacillus rhamnosus* derived from breast milk and use thereof.

**Background Art**

Probiotics are crucial for human growth and development as well as immune regulation. A large number of research literatures have proven that probiotics are closely related to gastrointestinal dysfunction (such as diarrhea, constipation, and inflammatory bowel disease), metabolic diseases (such as osteoporosis, obesity, hyperlipidemia, hypertension, diabetes, and hyperuricemia), and neurological diseases (such as insomnia, depression, Alzheimer's disease, and anxiety disorder).

At present, a wide variety of probiotics have been marketed. However, most probiotics are derived from intestinal flora or food. *Lacticaseibacillus rhamnosus* is one of the common probiotics. The National Health Commission of China has announced that there are 14 strains of probiotics that can be used in infant food in China, among which 3 strains are *Lacticaseibacillus rhamnosus,* two strains are derived from the intestinal tract and one strain from food. Due to limited sample sources and relatively low probiotic content, commercially available breast milk-derived probiotics account for a small proportion.

In view of this, the present application is specially proposed.

**Summary of the Invention**

Object of the Invention

The object of the present application is to provide a strain of *Lacticaseibacillus rhamnosus* derived from breast milk. This strain has good acid resistance, bile salt resistance, antibacterial properties and a good immune regulatory effect, and has promising application prospects in the fields of medicine and food.

Technical Solution

To achieve the object of the present application, the following technical solutions are provided:

In a first aspect, the present application provides a strain of *Lacticaseibacillus rhamnosus* B2-1 which is derived from breast milk. The *Lacticaseibacillus rhamnosus* was deposited in the China General Microbiological Culture Collection Center on December 8, 2022, with the deposit number CGMCC No. 26167.

In another aspect, the present application further provides use of the aforementioned *Lacticaseibacillus rhamnosus* B2-1 in the preparation of a medicament and/or a food.

Preferably or optionally, the medicament is used for immune regulation, or for the treatment, prevention, or alleviation of one or more of a gastrointestinal dysfunction, a metabolic disease, a neurological disease, and allergic rhinitis.

Alternatively, a method for preventing, alleviating, or treating at least one of the following diseases by using the aforementioned *Lacticaseibacillus rhamnosus* B2-1 is provided, which comprises administering an effective dose of the probiotic (*Lacticaseibacillus rhamnosus* B2-1) to a patient in need thereof.

The diseases include one or more of a gastrointestinal dysfunction, a metabolic disease, a neurological disease, and allergic rhinitis.

Preferably or optionally, the symptoms of the gastrointestinal dysfunction include at least one of diarrhea, constipation, and inflammatory bowel disease.

Preferably or optionally, the symptoms of the metabolic disease include at least one of osteoporosis, obesity, hyperlipidemia, hypertension, diabetes, and hyperuricemia; optionally, the treatment, prevention, or alleviation of the metabolic disease includes reducing uric acid.

Preferably or optionally, the neurological disease is at least one of insomnia, depression, Alzheimer's disease, and anxiety disorder.

Preferably or optionally, the food is a functional food used for immune regulation, for the alleviation of one or more symptoms of a gastrointestinal dysfunction, a metabolic disease, and a neurological disease.

Preferably or optionally, the symptoms of the gastrointestinal dysfunction include at least one of diarrhea, constipation, and inflammatory bowel disease.

Preferably or optionally, the symptoms of the metabolic disease include at least one of osteoporosis, obesity, hyperlipidemia, hypertension, diabetes and hyperuricemia.

Preferably or optionally, the neurological disease is at least one of insomnia, depression, Alzheimer's disease, and anxiety disorder.

In another aspect, the present application further provides a composition, which comprises the aforementioned *Lacticaseibacillus rhamnosus* B2-1 and/or a fermentation product thereof.

Beneficial effects

The *Lacticaseibacillus rhamnosus* provided by the present application is isolated from breast milk and is more easily accepted by consumers compared with lactobacilli from other sources. Meanwhile, this strain also has good acid resistance, bile salt resistance, antibacterial and immune regulatory properties. *Lacticaseibacillus rhamnosus* B2-1 significantly downregulates the expression of TNF$\alpha$, IL-1$\beta$, and IL-6 in spleen cells and upregulates the expression of IL-12. TNF$\alpha$, IL-1$\beta$, and IL-6 are important inflammatory factors, and IL-12 plays an important role in hypersensitivity reactions and has the effect of reducing uric acid. Therefore, *Lacticaseibacillus rhamnosus* B2-1 has good application prospects in the fields of medicine and food.

**Brief Description of the Drawings**

One or more embodiments are exemplarily illustrated by the corresponding figure(s) in the Drawings, and these exemplary illustrations do not constitute limitations to the embodiments. The term "exemplary" used exclusively herein means "serving as an example, embodiment, or illustrative". Any embodiment described herein as "exemplary" need not be construed as being superior to or better than other embodiments.

Figure 1 is a whole-genome diagram of *Lacticaseibacillus rhamnosus* B2-1;

Figure 2 is a result diagram of a 1-hour acid resistance performance experiment in Example 2;

Figure 3 is a result diagram of a 3-hour acid resistance performance experiment in Example 2;

Figure 4 is a result diagram of a bile salt resistance test in Example 3;

Figure 5 is a production curve diagram of *Lacticaseibacillus rhamnosus* B2-1 obtained in Example 4;

Figure 6 is a concentration diagram of TNF$\alpha$ for each group measured in Example 7;

Figure 7 is a concentration diagram of IL-1$\beta$ for each group measured in Example 7;

Figure 8 is a concentration diagram of IL-6 for each group measured in Example 7;

Figure 9 is a concentration diagram of IL-12 for each group measured in Example 7;

Figure 10 shows the bee hemolymph uric acid concentration for each group measured in Example 8; ** represents $P<0.01$, and *** represents $P<0.001$;

Figure 11 shows the Wnt_Rho1 gene expression in intestinal tissue for each group measured in Example 8; * represents $P<0.05$;

Figure 12 shows the Atg1 gene expression in intestinal tissue for each group measured in Example 8; *** represents $P<0.001$;

Figure 13 shows the MAPK_phl gene expression in intestinal tissue for each group measured in Example 8; *** represents $P<0.001$;

Figure 14 shows the PGRP_LC gene expression in intestinal tissue for each group measured in Example 8; ** represents $P<0.01$; and

Figure 15 shows the Toll gene expression in intestinal tissue measured in Example 8; * represents $P<0.05$, and ** represents $P<0.01$.

**Detailed Description of the Invention**

To make the objectives, technical solutions, and advantages of the embodiments of the present application clearer, the technical solutions in the examples of the present application are described clearly and completely below. Apparently, the described examples are part of the examples of the present application, rather than all the examples. Based on the examples in the present application, all other examples obtained by those of ordinary skill in the art without making creative efforts shall fall within the protection scope of the present application.

In addition, to better illustrate the present application, numerous specific details are provided in the following detailed description. Those skilled in the art should understand that the present application can also be implemented without certain specific details. In some examples, raw materials, elements, methods, means and the like well-known to those skilled in the art are not described in detail so as to highlight the gist of the present application.

Unless otherwise expressly stated, the term "including" or its variations such as "containing" or "comprising" throughout the description and claims shall be understood to include the stated elements or components, and shall not exclude other elements or components.

The present application provides a strain of *Lacticaseibacillus rhamnosus* B2-1, which was deposited in the China General Microbiological Culture Collection Center (Abbreviated as ACGMCC, Address: Institute of Microbiology, Chinese Academy of Sciences, No. 3, Courtyard 1, Beichen West Road, Chaoyang District, Beijing, Postcode: 100101) on December 8, 2022, with the deposit number CGMCC NO. 26167.

Herein, reagents and raw materials used in the examples of the present application are as follows: MRS/MRSA culture medium was purchased from Beijing Luqiao Technology Co., Ltd; mouse TNFα, IL-1β, IL-6, and IL-12 ELISA kits were purchased from Abcam company; and the remaining reagents are all commercially available.

The control commercial strain, *Lactobacillus rhamnosus* D001, was deposited on February 8, 2023 in the China General Microbiological Culture Collection Center (Abbreviated as CGMCC, Address: Institute of Microbiology, Chinese Academy of Sciences, No. 3, Courtyard 1, Beichen West Road, Chaoyang District, Beijing, Postcode: 100101), with the deposit number CGMCC No. 26533. MRS broth culture medium was purchased from Beijing Luqiao Technology Co., Ltd., with the article number CM187.

Nutritional agar was purchased from Beijing Luqiao Technology Co., Ltd., with the article number CM107.

*Escherichia coli* ATCC 25922 was purchased from Guangdong Microbial Strain Collection Center. *Staphylococcus aureus* ATCC 25923 was purchased from Guangdong Microbial Strain Collection Center.

*Shigella sonnei* ATCC 25931 was purchased from Guangdong Microbial Strain Collection Center. *Salmonella enteritidis* ATCC 14028 was purchased from Guangdong Microbial Strain Collection Center.

A preparation method for a strain supernatant used in each example of the present application is as follows: 100 μL of the corresponding strain cultured for 8 hours is inoculated in 10 mL of MRS broth culture medium, shake-cultured at 37°C and 220 rpm for 12 hours, and then centrifuged at 10000 rpm for 10 minutes, to obtain the supernatant.

Example 1

This example provided the isolation and identification of breast milk-derived *Lacticaseibacillus rhamnosus* B2-1.

A fresh breast milk sample was selected and gradient-diluted with sterile PBS buffer, then respectively spread on MRS solid medium containing 1% calcium carbonate and cultured under an anaerobic condition at 37°C for 48 hours. Well-grown single colonies were picked and amplified, followed by 16S sequencing. According to the sequencing result, the strain was named as *Lacticaseibacillus rhamnosus* B2-1 and subjected to enrichment culture in an anaerobic incubator at 37°C. After the culture was completed, it was amplified and preserved in laboratory and deposited in the China General Microbiological Culture Collection Center on December 8, 2022, with the deposit number CGMCC NO. 26167.

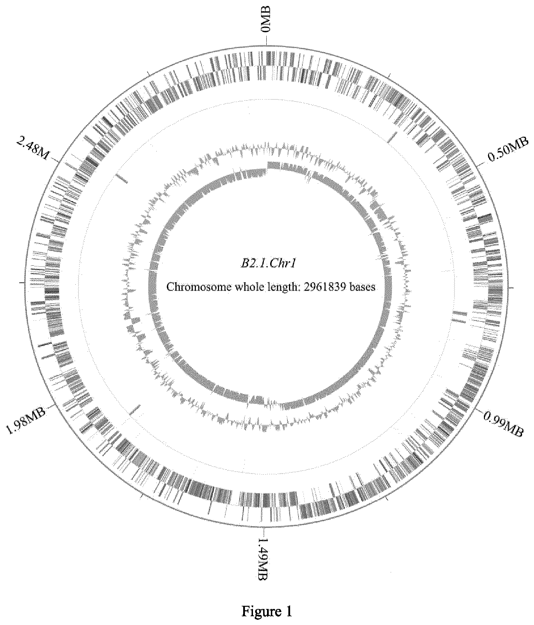

The B2-1 strain was identified as *Lacticaseibacillus rhamnosus* by the 16S sequencing. The whole-genome sequencing of the strain was performed by using Pacbio (10Kb SMRT Bell library) and Illumina PE150 (350bp small fragment library) sequencing platforms. The whole-genome diagram obtained by sequencing was shown in Figure 1, which demonstrates that *Lacticaseibacillus rhamnosus* B2-1 contains 2961839 bases and 2896 genes. The plasmid contains 30650 bases and 36 genes.

The sequencing results were annotated using the NR database.

Example 2

This example provided the acid resistance test of the aforementioned strain and a comparison with other strains.

*Lacticaseibacillus rhamnosus* B2-1, as well as the control *Lactobacillus rhamnosus* D001 (with the deposit number CGMCC NO. 26533) and *Lactobacillus plantarum* P6 (the deposit number of *Lactobacillus plantarum* P6 was CGMCC No. 19748, and this strain was disclosed in a patent application CN111778180A filed on June 12, 2020) were activated and then washed with sterile PBS buffer, respectively. Then they were respectively added to sterile MRS broth culture media with pH adjusted to 1.5, 2.0, or 3.0 using 1 mol/L hydrochloric acid, and anaerobically cultured at 37°C. Samples were taken at 0, 1, and 3 hours respectively. The samples were subjected to gradient dilution and spread on MRS solid medium plates, followed by anaerobic culture at 37°C for 48 hours. Colony counting was then performed, and the survival rate was calculated according to the following formula.

$$\text{Survival rate } (\%) = (N/N_{0h}) \times 100$$

wherein, N represents the colony count data at 1 hour or 3 hours, and $N_{0h}$ represents the colony count data at 0 hour.

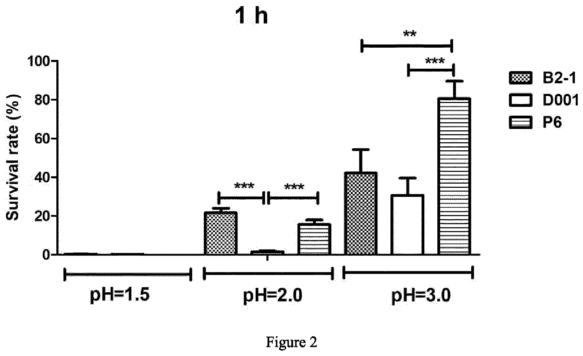

The results of acid resistance test were shown in Figures 2-3.

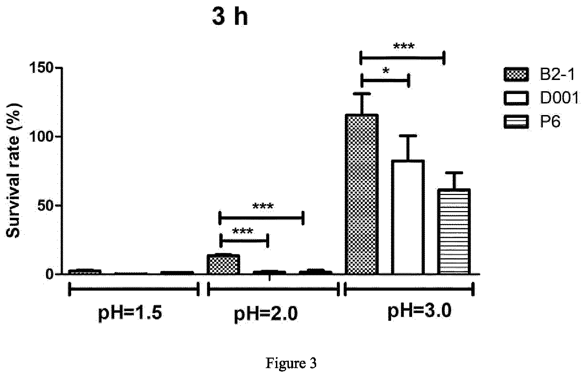

Probiotics need to pass through the action of gastric juice after entering the human body, and only the surviving probiotics can exert probiotic effects. As can be seen from Figures 2 and 3, *Lacticaseibacillus rhamnosus* B2-1, *Lactobacillus rhamnosus* D001, and *Lactobacillus plantarum* P6 were all unable to survive at pH 1.5 for 1 hour and 3 hours respectively. However, the survival rates of *Lacticaseibacillus rhamnosus* B2-1 at pH 2.0 for 1 hour and 3 hours were 21.45±2.45% and 13.41±0.81%, respectively; while the control strain *Lactobacillus rhamnosus* D001 was unable to survive at pH 2.0 for 1 hour and 3 hours; and P6 had a survival rate of 15.54±2.30% at pH 2.0 for 1 hour, but it was unable to survive at pH 2.0 for 3 hours. The survival rates of the strain B2-1 at pH 3.0 for 1 hour and 3 hours were 42.15±12.00% and 115.38±15.84%, respectively; the survival rates of the control strain D001 at pH 3.0 for 1 hour and 3 hours were 30.59±8.98% and 82.35±18.15%, respectively; and the survival rates of P6 at pH 3.0 for 1 hour and 3 hours were 80.36±8.75% and 61.43±19.01%, respectively. In conclusion, compared with the control strains, B2-1 had a better survival rate at pH 2.0. Its 3-hour survival rate at pH 3.0 was significantly higher than those of the control strains. The 3-hour survival rate of B2-1 at pH 3.0 was higher than its 1-hour survival rate, indicating that B2-1 had adapted to the pH 3.0 environment and started to proliferate. The above results indicated that the acid resistance of *Lacticaseibacillus rhamnosus* B2-1 was better than those of the control strains.

Example 3

This example provided tests on bile salt resistance of the aforementioned strain and a comparison with other strains.

*Lacticaseibacillus rhamnosus* B2-1, as well as control strains *Lactobacillus rhamnosus* D001 and *Lactobacillus plantarum* P6 (with the deposit number CGMCC No. 19748) were activated, and then washed with sterile PBS buffer, respectively. Then they were respectively resuspended in sterile MRS broth culture media containing 0.3% bile salts, and anaerobically cultured at 37°C. Samples were taken at 0, 1, and 3 hours, respectively. The samples were subjected to gradient dilution and spread on MRS solid medium plates, followed by anaerobic culture at 37°C for 48 hours. Colony counting was then performed, and the survival rate was calculated according to the following formula:

$$\text{Survival rate (\%)} = (N/N_{0h}) \times 100$$

wherein, N represents the colony count data at 1 hour or 3 hours, and $N_{0h}$ represents the colony count data at 0 hour.

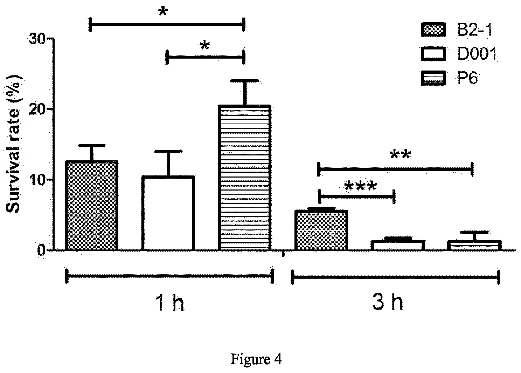

The results of bile salt resistance test were shown in Figure 4.

As shown in Figure 4, after 1 hour of culture in MRS broth containing 0.3% bile salts, the survival rate of the strain B2-1 was 12.54±2.36%, while the survival rates of control strains D001 and P6 were 10.39±3.61% and 20.43±4.12%, respectively. The survival rate of the control strain P6 was significantly higher than those of B2-1 and D001. After 3 hours of culture, the survival rate of B2-1 decreased to approximately 5%, whereas the survival rates of D001 and P6 decreased more significantly, with only about 1% survival. In conclusion, when cultured in MRS broth containing 0.3% bile salts, the 3-hour survival rate of B2-1 was significantly higher than those of control strains D001 and P6, indicating that the strain B2-1 has good bile salt resistance.

Example 4

This example provided a growth curve of the strain *Lacticaseibacillus rhamnosus* B2-1.

The activated strain B2-1 was inoculated into MRS broth culture medium and placed in a 37°C incubator for anaerobic culture. The absorbance $OD_{600}$ was measured at 0, 2, 4, 6, 8, 10, 12, 16, 20, 24, 28, 32, 36, 40, and 48 hours, respectively, and a growth curve was plotted.

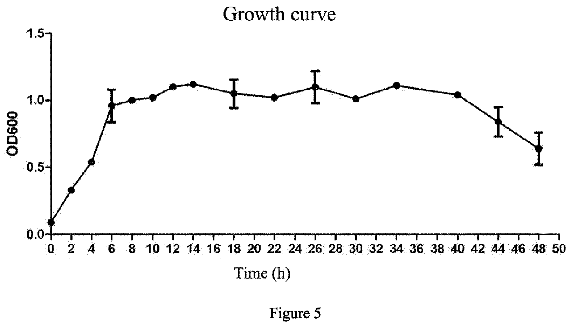

The plotted growth curve was shown in Figure 5.

As shown in Figure 5, the strain B2-1 had a relatively short lag phase, and entered a logarithmic phase within 2-6 hours. After 6 hours of culture, it entered a stationary phase and reached a higher bacteria concentration. It remained in the stationary phase from 8 to 40 hours of culture and began to enter a decline phase after 40 hours.

Example 5

This example provided tests of antibacterial activity of the aforementioned strain and a comparison with other strains.

*Escherichia coli* ATCC25922, *Staphylococcus aureus* ATCC25923, *Shigella sonnei* ATCC25931, and *Salmonella enteritidis* ATCC14028 were used as indicator bacteria. The activated indicator bacteria were spread on nutritional agar, and their antibacterial activity was preliminarily determined using an agar diffusion method. Oxford cups were placed on plates using a sterile tweezer, and sterile nutritional agar was poured to fix the Oxford cups. After the agar was solidified, the Oxford cups were removed. 100 μL of the prepared supernatants of B2-1, D001, and P6 were pipetted and added to the wells, respectively. The plates were placed in a refrigerator at 4°C for diffusion for approximately 3 hours, and then incubated at 37°C for 24 hours. The diameters of the inhibition zones were observed and recorded. The results were expressed as mean ± standard deviation.

The antibacterial activity test results were shown in Table 1.

Table 1 Antibacterial activity test results

| Strain name | *Escherichia coli* ATCC 25922 | *Staphylococcus aureus* ATCC 25923 | *Shigella sonnei* ATCC25931 | *Salmonella enteritidis* ATCC14028 |
|---|---|---|---|---|
| | Inhibition zone diameter (mm) | | | |
| B2-1 | 22.78±1.67 | 16.41±2.65 | 25.00±3.02 | 23.89±2.07 |
| D001 | 20.56±1.58 | 22.66±2.44 | / | / |
| P6 | 29.30±3.52 | 23.52±3.50 | 26.97±2.84 | 26.56±3.98 |

Probiotics might come into contact with pathogenic bacteria in the body, and therefore probiotics require a certain antibacterial activity to exert their effects in human body. As shown in Table 1, the strain B2-1 had a certain inhibitory ability against *Escherichia coli* ATCC25922, *Staphylococcus aureus* ATCC25923, *Shigella sonnei* ATCC25931, and *Salmonella enteritidis* ATCC14028. In contrast, D001 showed a certain inhibitory activity only against *Escherichia coli* ATCC25922 and *Staphylococcus aureus* ATCC25923. That is to say, B2-1 has superior comprehensive antibacterial performance compared to the strain D001 of the same species.

Example 6

This example provided antibiotic resistance testing of the strain *Lacticaseibacillus rhamnosus B2*-1.

Third-generation B2-1 bacteria solution was diluted to a concentration of $10^7$ CFU/mL. 100 μL of the diluted bacteria solution was evenly spread on MRS solid culture medium using a sterile spreader. After incubating for 20 minutes, antibiotic susceptibility discs and a blank control disc were placed equidistantly on the MRS solid culture medium, and gently pressed to ensure adhesion. Following anaerobic culture at 37°C for 18-24 hours, the diameter (mm) of each inhibition zone was measured using a vernier caliper. The results were expressed as mean ± standard deviation. The antibiotic susceptibility discs used in this example included 6 antibiotics: ceftriaxone, tetracycline, penicillin, gentamicin, enrofloxacin, and polymyxin B.

The antibiotic resistance testing results were shown in Table 2.

Table 2 Antibiotic resistance testing results of the strain *Lacticaseibacillus rhamnosus* B2-1

| Serial No. | Antibiotic Class | Antibiotic Name | Drug Content per Disc | Susceptibility Zone Diameter (mm) | Result Interpretation |
|---|---|---|---|---|---|
| 1 | Cephalosporins | Ceftriaxone | 30μg | 30.55±4.58 | S |
| 2 | Tetracyclines | Tetracycline | 30μg | 9.95±0.88 | R |
| 3 | Penicillins | Penicillin | 10U | 27.44±2.44 | S |
| 4 | Aminoglycosides | Gentamicin | 10μg | 26.54±2.15 | S |
| 5 | Fluoroquinolones | Enrofloxacin | 10μg | 10.30±1.68 | R |
| 6 | Polypeptides | Polymyxin B | 300IU | / | R |

From Table 2, it can be seen that the strain B2-1 was sensitive to ceftriaxone, penicillin, and gentamicin, while resistant to tetracycline, enrofloxacin, and polymyxin B.

Example 7

This example provided the immune regulation performance testing of the aforementioned strain and a comparison with other strains.

C57BL/6 mice aged 6-8 weeks were and euthanized by cervical dislocation, soaked in 75% ethanol for 3 minutes, and then placed on sterile filter paper. Spleens were harvested and placed in a culture dish containing 5 mL of Hanks' solution. The spleens were placed on a sterile 100-mesh sieve and homogenized through the sieve using a sterile pestle to obtain a mouse splenocyte suspension. The concentration of the prepared mouse splenocyte suspension was adjusted to $1\times10^6$ cells/mL for later use.

A 96-well plate was taken and 100 μL of the previously prepared mouse splenocyte suspension was added to each well. Then, 20 μL of the supernatant of B2-1, D001, or P6 was added to the respective wells. Lipopolysaccharide (LPS) and concanavalin A (ConA) were added to each well to achieve final concentrations 500 μg/mL for LPS and 250 μg/mL for ConA. A blank model group was set up by replacing the supernatant with an equal volume of Hanks' solution. All plates were incubated for 48 hours in an environment of 37°C with 5% $CO_2$.

Supernatants were collected from the respective wells, and TNFα, IL-1β, IL-6, and IL-12 ELISA kits were respectively used to detect the concentrations of corresponding immune factors in the supernatants.

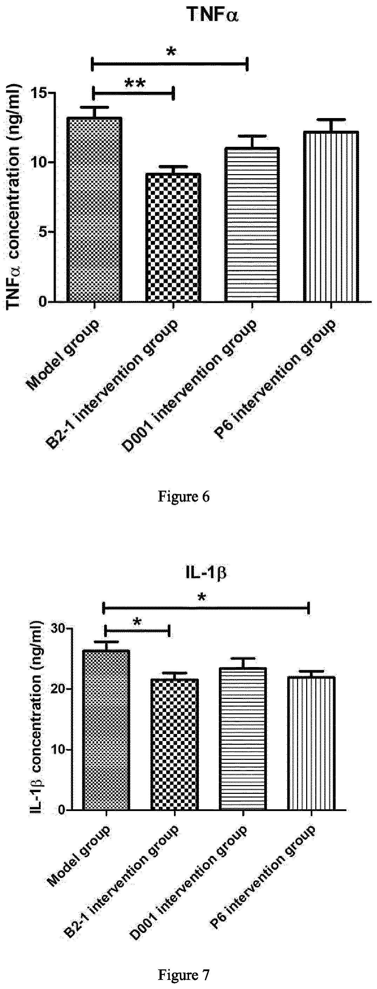

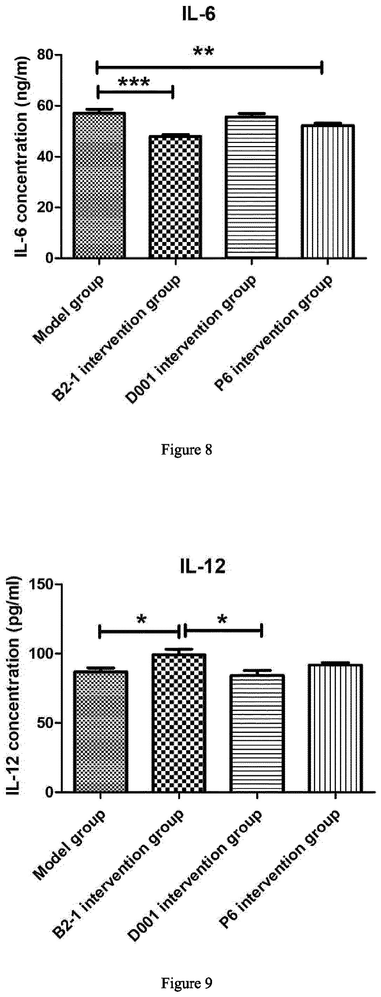

The measurement results were shown in Figures 6-9.

Immune regulation is a critical probiotic function, as many chronic and metabolic diseases are closely associated with immune dysfunction. In this example, mouse splenocytes were stimulated with LPS and ConA, followed by co-incubation with the culture supernatants of the respective strains for 48 hours. As shown in Figures 6-9, *Lacticaseibacillus rhamnosus* B2-1 significantly downregulated the concentrations of TNF-α, IL-1β, and IL-6 in the splenocyte culture supernatant and upregulated the level of IL-12. In contrast, the strain D001 (also a strain of *Lacticaseibacillus rhamnosus*) only downregulated the level of TNF-α and exhibited no regulatory effect on other inflammatory factors. *Lactobacillus plantarum* P6 downregulated IL-1β and IL-6 concentrations but had no effect on TNF-α or IL-12. These in vitro cell experiments demonstrate that *Lacticaseibacillus rhamnosus* B2-1 has strong immune regulatory activity and significant probiotic potential.

Example 8

Strains and animals: *Lacticaseibacillus rhamnosus* B2-1 isolated in Example 1, and honey bees (*Apis mellifera*) purchased from China Agricultural University. This experiment was entrusted to Suzhou Fenghua Biotechnology Co., Ltd.

Experimental methods

1) Grouping: 120 1-day-old bees were assigned to each of a blank group, a model group, and an intervention group.
2) Hyperuricemia modeling and probiotic intervention. Modeling: 120 1-day-old bees in the blank group were fed with 50% sugar water plus milk powder for 12 days; in addition, 240 1-day-old bees were subjected to hyperuricemia modeling by being fed with 50% sugar water plus milk powder plus 1% yeast-derived RNA for 7 days. The blood uric acid level of the model group (bee blood uric acid refers to uric acid in bee hemolymph, which is equivalent to uric acid in the human blood) increased significantly, indicating that the hyperuricemia model was successfully established. These bees were randomly divided into the model group and the intervention group (240 bees in each of the model group and the intervention group). Bees in both the model group and the intervention group continued to be fed with hyperuricemia modeling bee food. Meanwhile, the intervention group was fed with a probiotic sample daily, and supplemented at $10^{10}$ CFU/g (probiotic powder and pollen were added to the bee food at a ratio of 1:20, and the diet was replaced every 24 hours to maintain probiotic activity). Uric acid evaluation was performed after 5 days of probiotic nutritional intervention, to compare blood uric acid levels and inflammatory factor gene expression indicators among the control group, the model group, and the intervention group.

The bee hyperuricemia model can be obtained using the above modeling method. In mammals such as rats/mice, it is not easy to obtain a stable chronic hyperuricemia model due to the presence of uricase. It has been reported in the literature that a stable hyperuricemia model can be relatively easily obtained using fruit flies. We used *Apis mellifera* for hyperuricemia modeling and also obtained a stable hyperuricemia model. Compared with mammals, the bee hyperuricemia modeling has a short cycle, low cost, large sample size, and stable hyperuricemia level. For the specific modeling method, please refer to the following references:

①), Tyler A U Hilsabeck, Ru Liu-Bryan, Tracy Guo, et al. A fly GWAS for purine metabolites identifies human FAM214 homolog medusa, which acts in a conserved manner to enhance hyperuricemia-driven pathologies by modulating purine metabolism and the inflammatory response. Geroscience. 2022 Aug;44(4):2195-2211. doi: 10.1007/s11357-022-00557-9.
②), Pawel Migdal, Agnieszka Murawska, Ewelina Berbeć, et al. Selected Biochemical Markers Change after Oral Administration of Pesticide Mixtures in Honey Bees. Toxics. 2022 Oct 5;10(10):590. doi: 10.3390/toxics10100590.
③), Yang Tong, Yifeng Wei, Yingjie Ju, et al. Anaerobic purinolytic enzymes enable dietary purine clearance by engineered gut bacteria. Cell Chem Biol. 2023 Sep 21;30(9):1104-1114.e7. doi: 10.1016/j.chembiol.2023.04.008.

④), Jin Mengjie, Li Zhen, Liu Jianhui, et al. Comparison of morphological, physiological, and biochemical indicators between Apis mellifera eclosion out-of-hive queen bees and worker bees. Journal of Jiangxi Agricultural University 2023, 45(5). DOI: 10.13836/j.jjau.2023112.

3) mRNA expression of inflammatory factors: Bee intestinal tissues were collected, quick-frozen in liquid nitrogen, and stored in a refrigerator at -80°C. After retrieval, RNA was extracted from the intestinal tissues and reverse-transcribed to obtain a sample cDNA. The gene expression levels of immune factors including Wnt_Rho1, Atg1, MAPK_phl, PGRP-LC, and Toll were detected by qPCR.

Experimental results:

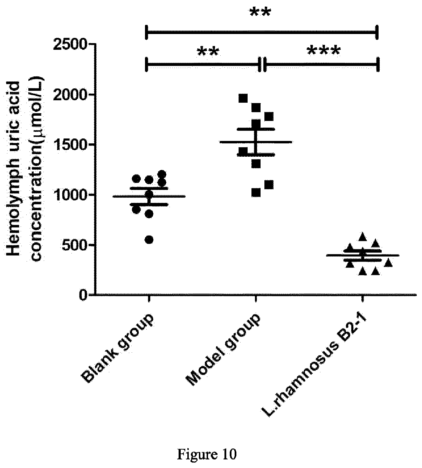

The hemolymph uric acid levels of bees in each group were shown in Figure 10. The results indicated that the hemolymph uric acid concentration of normal bees in the blank group was $981.91 \pm 226.84$ μmol/L, while that in the model group was $1523.70 \pm 357.42$ μmol/L. The model group was significantly higher than the blank group (P<0.01), demonstrating the successful establishment of the bee hyperuricemia model. After intervention with *Lacticaseibacillus rhamnosus* B2-1, the concentration of the hemolymph uric acid decreased to $394.55 \pm 130.11$ μmol/L, which was not only significantly lower than that of the model group (P<0.001), but also significantly lower than that of the blank group (P<0.01).

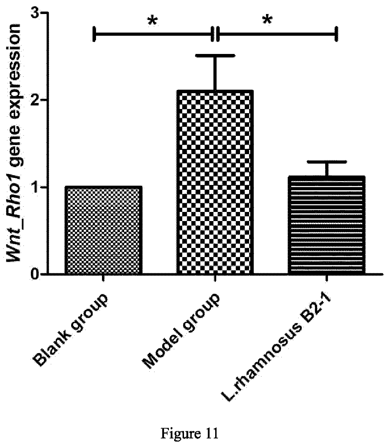

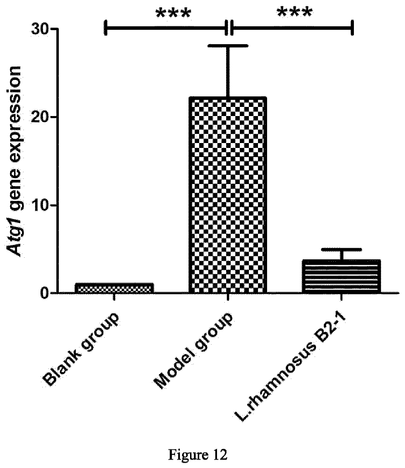

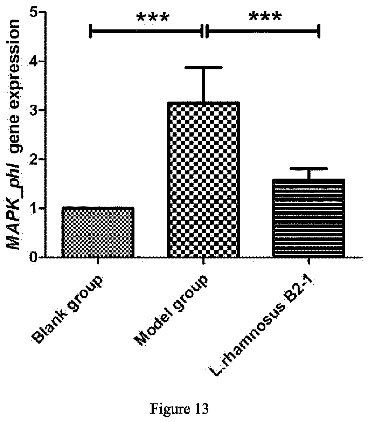

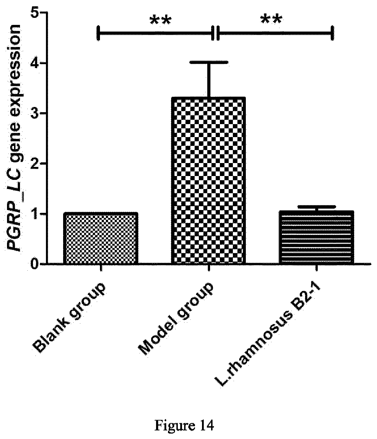

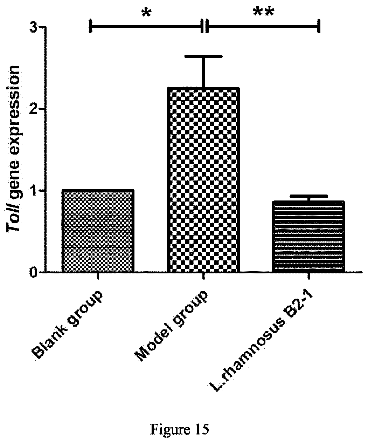

mRNA detection was performed on the bees, and results were shown in Figures 11~15. It was found from the results that the mRNA levels of key factors related to the inflammatory pathways in the model group was significantly increased, for example, Wnt_Rho1 was upregulated by 2-fold (Figure 11), Atg1 gene expression was upregulated by more than 20-fold (Figure 12), MAPK_phl and PGRP_LC gene expressions were upregulated by 3-fold (Figures 13 and 14), and Toll gene expression was upregulated by 2-fold (Figure 15). After the intervention with *Lacticaseibacillus rhamnosus* B2-1, these gene expressions were significantly downregulated and restored to the level of the blank group. These results indicated that *Lacticaseibacillus rhamnosus* B2-1 can not only significantly reduce the level of hemolymph uric acid in bees but also regulate immune pathways and decrease the expression of inflammatory factors.

As can be seen from the above examples, *Lacticaseibacillus rhamnosus* B2-1 provided by this application has good acid resistance and bile salt resistance. *In vitro* experiments have also confirmed that it has excellent pathogenic bacteria-inhibiting and immune-regulating functions. Therefore, it has promising application prospects, especially as a drug for treating one or more of gastrointestinal dysfunction, metabolic diseases, and neurological diseases, and as a functional food for alleviating one or more symptoms of gastrointestinal dysfunction, metabolic diseases, and neurological diseases.

Finally, it should be noted that the above examples are only used to illustrate the technical solutions of this application, rather than limiting them; although the present application has been described in detail with reference to the aforementioned examples, those of ordinary skill in the art should understand that they can still modify the technical solutions recited in the aforementioned examples, or equivalently replace some of the technical features therein; and these modifications or replacements do not make the essence of the corresponding technical solutions deviate from the spirit and scope of the technical solutions of each example of this application.

Industrial Applicability

The present application provides a breast milk-derived *Lacticaseibacillus rhamnosus* and use thereof. The *Lacticaseibacillus rhamnosus* was deposited on December 8, 2022 in the China General Microbiological Culture Collection Center with the deposit number CGMCC NO. 26167. The present application further provides use of the above *Lacticaseibacillus rhamnosus* in the preparation of a medicament and/or a food, as well as a composition comprising the *Lacticaseibacillus rhamnosus* and/or a fermentation product thereof. The *Lacticaseibacillus rhamnosus* of the present application is isolated from breast milk and is more easily accepted by consumers compared with lactobacillus from other sources; at the same time, this strain also exhibits good acid resistance, bile salt resistance, antibacterial activity, and immune regulatory properties, and thus has promising application prospects in the fields of medicine and food.

**Claims**

1. A breast milk-derived *Lacticaseibacillus rhamnosus* B2-1, wherein the *Lacticaseibacillus rhamnosus* was deposited on December 8, 2022 in the China General Microbiological Culture Collection Center with the deposit number CGMCC No. 26167.

2. Use of the *Lacticaseibacillus rhamnosus* B2-1 according to claim 1 in the preparation of a medicament and/or a food.

3. The use according to claim 2, wherein that the medicament is used for immune regulation, or for the treatment, prevention, or alleviation of one or more of a gastrointestinal dysfunction, a metabolic disease, a neurological disease, and allergic rhinitis.

4. The use according to claim 3, wherein the symptoms of the gastrointestinal dysfunction include at least one of diarrhea, constipation, and inflammatory bowel disease.

5. The use according to claim 3, wherein the symptoms of the metabolic disease include at least one of osteoporosis, obesity, hyperlipidemia, hypertension, diabetes, and hyperuricemia; optionally, the treatment, prevention, or alleviation of the metabolic disease comprises reducing uric acid.

6. The use according to claim 3, wherein the neurological disease is at least one of insomnia, depression, Alzheimer's disease, and anxiety disorder.

7. The use according to claim 2, wherein the food is a functional food used for immune regulation, or for the alleviation of one or more symptoms of a gastrointestinal dysfunction, a metabolic disease, and a neurological disease.

8. The use according to claim 7, wherein that the symptoms of the gastrointestinal dysfunction include at least one of diarrhea, constipation, and inflammatory bowel disease.

9. The use according to claim 7, wherein the symptoms of the metabolic disease include at least one of osteoporosis, obesity, hyperlipidemia, hypertension, diabetes, and hyperuricemia.

10. The use according to claim 7, wherein the neurological disease is at least one of insomnia, depression, Alzheimer's disease, and anxiety disorder.

11. A composition, which comprises the *Lacticaseibacillus rhamnosus* B2-1 according to claim 1 and/or a fermentation product thereof.

B2.1.Chr1

Chromosome whole length: 2961839 bases

Figure 1

Figure 2

Figure 3

Figure 4

Figure 5

# TNFα

Figure 6

# IL-1β

Figure 7

Figure 8

Figure 9

Figure 10

Figure 11

Figure 12

Figure 13

Figure 14

Figure 15

## INTERNATIONAL SEARCH REPORT

| International application No. |
|---|
| **PCT/CN2024/074770** |

| A. | CLASSIFICATION OF SUBJECT MATTER |
|---|---|

C12N 1/20(2006.01)i; A61K 35/747(2015.01)i; A61P 31/04(2006.01)i; A23L 33/135(2016.01)i; C12R 1/225(2006.01)i

According to International Patent Classification (IPC) or to both national classification and IPC

| B. | FIELDS SEARCHED |
|---|---|

Minimum documentation searched (classification system followed by classification symbols)

IPC: C12N, A61K, A61P, A23L, C12R

Documentation searched other than minimum documentation to the extent that such documents are included in the fields searched

Electronic data base consulted during the international search (name of data base and, where practicable, search terms used)

VEN, CNABS, CNTXT, EPTXT, USTXT, WOTXT, ISI Web of Knowledge, CNKI: 鼠李糖乳酪杆菌, 耐酸, 耐胆盐, 抑菌, 大肠杆菌, 金黄色葡萄球菌, 宋氏志贺氏菌, 肠炎沙门氏菌, 免疫调节, Lacticaseibacillus rhamnosus, CGMCC NO.26167, acid, cholate, resistance, inhibition, staphylococcus aureus, escherichia coli, salmonella enteritidis, shigella sonnei, immunoregulation, TNFα, IL-1β, IL-6, IL-12

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 110122877 A (SHENZHEN BGI AGRICULTURE APPLICATION RESEARCH INSTITUTE et al.) 16 August 2019 (2019-08-16) abstract, claims 1-2, and description, paragraphs 17, 40, 54, and 98 | 1-11 |
| A | CN 112210507 A (JIANGSU WECARE BIOTECHNOLOGY CO., LTD.) 12 January 2021 (2021-01-12) abstract, and description, paragraphs 23, 34, 79, and 92 | 1-11 |
| A | CN 111944721 A (NORTHEAST AGRICULTURAL UNIVERSITY) 17 November 2020 (2020-11-17) entire document | 1-11 |
| A | CN 114259056 A (MICROHEALTH PROBIOTIC (SUZHOU) STOCK LIMITED COMPANY) 01 April 2022 (2022-04-01) entire document | 1-11 |
| A | US 2022296499 A1 (ELIGO BIOSCIENCE) 22 September 2022 (2022-09-22) entire document | 1-11 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 April 2024** | **09 May 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/074770** |

### C. DOCUMENTS CONSIDERED TO BE RELEVANT

| Category* | Citation of document, with indication, where appropriate, of the relevant passages | Relevant to claim No. |
| --- | --- | --- |
| A | WO 2022128927 A1 (DUPONT NUTRITION BIOSCIENCES APS) 23 June 2022 (2022-06-23)<br>    entire document | 1-11 |
| A | PENG, Xianqi et al. "Lacticaseibacillus Rhamnosus Alleviates Intestinal Inflammation and Promotes Microbiota-Mediated Protection against Salmonella Fatal Infections"<br>*Frontiers in Immunology,* Vol. 13, 11 August 2022 (2022-08-11), 973224<br>    entire document | 1-11 |

International application No.

**PCT/CN2024/074770**

| Patent document cited in search report | | | Publication date (day/month/year) | Patent family member(s) | | | Publication date (day/month/year) |
|---|---|---|---|---|---|---|---|
| CN | 110122877 | A | 16 August 2019 | None | | | |
| CN | 112210507 | A | 12 January 2021 | None | | | |
| CN | 111944721 | A | 17 November 2020 | None | | | |
| CN | 114259056 | A | 01 April 2022 | None | | | |
| US | 2022296499 | A1 | 22 September 2022 | US | 2023218505 | A1 | 13 July 2023 |
| | | | | WO | 2022195108 | A2 | 22 September 2022 |
| | | | | WO | 2022195108 | A3 | 10 November 2022 |
| | | | | US | 11633348 | B2 | 25 April 2023 |
| WO | 2022128927 | A1 | 23 June 2022 | EP | 4014755 | A1 | 22 June 2022 |
| | | | | EP | 4262437 | A1 | 25 October 2023 |
| | | | | KR | 20230118939 | A | 14 August 2023 |
| | | | | CA | 3201727 | A1 | 23 June 2022 |
| | | | | AU | 2021399012 | A1 | 22 June 2023 |
| | | | | JP | 2023552860 | A | 19 December 2023 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310050530

- CN 111778180 A

**Non-patent literature cited in the description**

- **TYLER A U HILSABECK** ; **RU LIU-BRYAN** ; **TRACY GUO et al.** A fly GWAS for purine metabolites identifies human FAM214 homolog medusa, which acts in a conserved manner to enhance hyperuricemia-driven pathologies by modulating purine metabolism and the inflammatory response.. *Geroscience.*, August 2022, vol. 44 (4), 2195-2211

- **YANG TONG** ; **YIFENG WEI** ; **YINGJIE JU et al.** Anaerobic purinolytic enzymes enable dietary purine clearance by engineered gut bacteria.. *Cell Chem Biol.*, 21 September 2023, vol. 30 (9), 1104-1114

- **JIN MENGJIE** ; **LI ZHEN** ; **LIU JIANHUI et al.** Comparison of morphological, physiological, and biochemical indicators between Apis mellifera eclosion out-of-hive queen bees and worker bees.. *Journal of Jiangxi Agricultural University*, 2023, vol. 45 (5)